# EUROPEAN PATENT SPECIFICATION

(11) **EP 3 548 059 B1**
(45) Date of publication and mention of the grant of the patent: **21.07.2021**
(21) Application number: 18704560.4
(22) Date of filing: 14.02.2018
(51) Int. Cl.: A61K 38/15, A61K 31/7036, A61P 31/04

(54) **COMBINATIONS OF LYSOBACTIN AND AMINOGLYCOSIDES AGAINST DISEASES CAUSED BY GRAM-POSITIVE AND GRAM-NEGATIVE BACTERIA IN NON-HUMAN ANIMALS**
KOMBINATIONEN VON LYSOBACTIN UND AMINOGLYCOSIDEN GEGEN KRANKHEITEN, DIE DURCH GRAM-POSITIVE UND GRAM-NEGATIVE BAKTERIEN IN NICHTHUMANEN TIEREN VERURSACHT SIND
COMBINAISONS DE LYSOBACTINE ET D'AMINOGLYCOSIDES CONTRE DES MALADIES PROVOQUÉES PAR DES BACTÉRIES À GRAM POSITIF ET À GRAM NÉGATIF CHEZ DES ANIMAUX NON HUMAINS

(30) Priority: 17.02.2017 EP 17156737
(43) Date of publication of application: 09.10.2019
(73) Proprietor: Bayer Animal Health GmbH, 51373 Leverkusen (DE)
(72) Inventor: SCHIFFER, Guido, 42111 Wuppertal (DE); FÄLKER, Stefan, 50676 Köln (DE); DAUBE, Gert, 51766 Engelskirchen (DE); WIEHL, Wolfgang, 50999 Köln (DE); KÖBBERLING, Johannes, 41466 Neuss (DE)
(74) Representative: Tier 1 Patents
(86) International application number: PCT/EP2018/053667
(87) International publication number: WO 2018/149871

(56) References cited:
- US-A1- 2006 264 358
- J.P. GANIÈRE ET AL: "Synergistic interactions between cefalexin and kanamycin in Mueller-Hinton broth medium and in milk", JOURNAL OF APPLIED MICROBIOLOGY., vol. 107, no. 1, 1 July 2009 (2009-07-01), pages 117-125, XP055376865, GB ISSN: 1364-5072, DOI: 10.1111/j.1365-2672.2009.04191.x
- O'SULLIVAN J ET AL: "LYSOBACTIN, A NOVEL ANTIBACTERIAL AGENT PRODUCED BY LYSOBACTER SP. I. TAXONOMY, ISOLATION AND PARTIAL CHARACTERIZATION", THE JOURNAL OF ANTIBIO, NATURE PUBLISHING GROUP, GB, no. 12, 1 December 1988 (1988-12-01), pages 1740-1744, XP009059528, ISSN: 0021-8820 cited in the application

## Description

The present invention relates to the combination of lysobactin with an aminoglycoside antibiotic for use in the treatment of diseases caused by Gram-positive and Gram-negative bacteria in non-human animals. The invention proved to be particularly useful relating to the treatment of mastitis in non-human animals. Particularly bovine mastitis can be well addressed by the present invention.

Mastitis is the inflammation of udder tissue and continues to be the most frequent and costly disease of dairy cattle. Financial losses due to mastitis occur for both subclinical and clinical stages of the disease. Losses caused by subclinical mastitis are well documented. Each doubling of the somatic cell count (SCC) above 50,000 cells/ml results in a loss of 0.4 kg and 0.6 kg of milk per day in first lactation and older cows, respectively (Hortet P, H. Seegers. 1998. Calculated milk production losses associated with elevated somatic cell counts in dairy cows: review and critical discussion. Vet Res. 29(6):497-510). Losses caused by clinical mastitis include discarded milk, transient reductions in milk yield and premature culling. Treatment of mastitis should be targeted towards the causative bacteria whenever possible.

Pyörälä S in Irish Veterinary Journal, Volume 62 Supplement 40-44 2009 has the following suggestions for antimicrobial treatment of clinical mastitis due to different pathogens:

| **Microorganism** | **Species** | **Drug of choice** | **Alternative** |
|---|---|---|---|
| Streptococci | *Streptococcus agalacticae Streptococcus dysgalacticae Streptococcus uberis Enterococci* | Penicillin G | |
| | | According to susceptibility testing | |
| Staphylococci | *Staphylococcus aureus* Coagulase negative staphylococci β-lactamase -ve *Staphylococcus aureus* Coagulase negative staphylococci β-lactamase +ve | Penicillin G | |
| | | No antimicrobials | Cloxacillin Macrolides Lincosamides |
| Coliforms | *Escherichia coli Klebsiella spp.* | No antimicrobials | Fluoroquinolones Cephalosporins |

A recent pharmaceutical composition for the treatment of mastitis in dairy cows is Ubrolexin®, a broad spectrum antibiotic against both Gram-positive and Gram-negative mastitis-causing bacteria. Ubrolexin® is a combination of cefalexin and kanamycin and is indicated for the treatment of clinical mastitis due to *Staphylococcus aureus, Streptococcus dysgalacticae, Streptococcus uberis* and *Escherichia coli.* The mode of action for cefalexin, which is an antibiotic of the cephalosporin group of substances, is an irreversible binding to D-alanine transpeptidase which disrupts the bacterial cell wall synthesis. Kanamycin, which is an aminoglycoside antibiotic, acts by attaching to the 30S subunit of membrane associated ribosomes and inhibits or interferes with bacterial protein synthesis.

Accordingly the combination of antibiotics, and in particular of aminoglycosides with other antibiotic substances is known per se.

A reoccurring problem with antibiotics in the prior art is that the pathogens may develop resistance towards the drugs employed in the treatment of the diseases they cause. Therefore, new active pharmaceutical ingredients are constantly sought after. The issue of developing resistances is even worsened when combinational use is made of antibiotic substances that do not synergize, but only target different pathogens. In those cases the pathogens that are not the main target of the first antibiotic may successively develop a full resistance against such first antibiotic.

Lysobactin was first isolated from the fermentation broth of *Lysobacter sp.* SC-14076 (ATCC 53042). Independently, scientists discovered katanosin A and B from a soil bacterium related to the genus Cytophaga (producer strain PBJ-5356). Katanosin B turned out to be identical to lysobactin. Bacterial cell wall biosynthesis is the primary target of these antibiotics; however, the mechanism of action is different from that of vancomycin. The lysobactins are cyclic depsipetides containing several nonproteinogenic D- and L-amino acids.

The natural product lysobactin and some derivatives are described as having antibacterial activity in US 4,754,018. The isolation and antibacterial activity of lysobactin is also described in EP 196 042 A1 and JP 01-132600.

The antibacterial activity of lysobactin and katanosin A is furthermore described in O'Sullivan, J. et al., J. Antibiot. (1988) 41 :1740-1744, Bonner, D. P. etal., J. Antibiot. (1988) 41:1745-1751, Shoji, J. etal., J. Antibiot. (1988) 41:713-718 and Tymiak,A. A. etal., J. Org. Chem. (1989) 54:1149-1157.

In contrast to the modes of action for cefalexin and kanamycin, lysobactin has been suggested to directly interact with the bacterial cell wall precursor lipid II (Lee, W. et al, Am. Chem. Soc. (2016) 138(1):100-113 and Breukink E. and de Kruijff B., Nat. Rev. Drug Disc. (2006) 5:321-323). Derivatives of lysobactin are the subject of the patent applications WO 2004/099239 A1, WO 2006/042653 A1, WO 2006/042654 A1, WO 2006/042655 A1, WO 2006/048139 A1, WO 2006/048140 A1, WO 2006/048156 A1, WO 2007/085456 A1, WO 2007/118691 A1 and WO 2007/118693 A1.

J.P. Ganiére et. al., Journal of Applied Microbiology 107 (2009), 117-125 describe the synergistic effect of cephalexin and kanamycin on the *in vitro* bactericidal activity against *Streptococcus uberis, Staphylococcus aureus* and *Escherichia coli* strains isolated from field cases of bovine mastitis.

None of the prior art listed is concerned with the treatment of bovine mastitis using lysobactin. Particularly, there is no prior art on the combined use of lysobactin with other antibiotic substances for the treatment of mastitis. Particularly the treatment of bovine mastitis being a major cause of commercial farming losses lacks suitable options in the art.

While it has recently been found in the international patent application number PCT/EP2016/069380 that the use of lysobactin alone is surprisingly highly beneficial in the treatment of bovine mastitis that is mainly caused by Gram-positive pathogens, particularly *Staphylococcus* bacteria, *Streptococcus* bacteria, *Trueperella* bacteria and/or *Corynebacterium* bacteria, because of its resistance breaking properties, there remains uncertainty on the efficiency of lysobactin as a potential agent against Gram-negative pathogens and a potential synergy with other antibiotic substances.

Accordingly, there remains a need in the art for a - particularly bovine - mastitis treatment that does not suffer from the drawback of bacterial resistance towards the active ingredients and that can synergistically address Gram-positive and Gram-negative pathogens. The present invention has the object of providing a solution for that. Accordingly, the present invention is particularly directed towards the combination of lysobactin with at least one aminoglycoside antibiotic for use in the treatment of mastitis. Especially the treatment of bovine mastitis as the commercially most relevant disease of this type is targeted by the present invention. It has surprisingly been found that lysobactin combined with aminoglycoside antibiotics synergize to result in extending antibacterial properties on both Gram-positive and Gram-negative pathogens associated with diseases caused by those pathogens in non-human animals. The effect of such synergy is particularly evident in female animals suffering from mastitis caused by such pathogens, while bovine mastitis turned out to be the most commercially relevant application of the herein described invention.

In the context of the present invention, the term "lysobactin" refers to the substance with the CAS number 118374-47-3 and its physiologically acceptable salts. Lysobactin has the following structure:

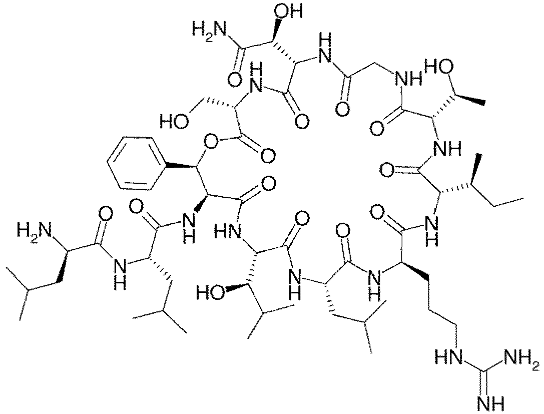

The bovine mastitis to be addressed is in particular the mastitis of dairy cattle.

Physiologically acceptable salts of lysobactin include acid addition salts of mineral acids, carboxylic acids and sulphonic acids, e.g. salts of hydrochloric acid, hydrobromic acid, sulphuric acid, phosphoric acid, methanesulphonic acid, ethanesulphonic acid, toluenesulphonic acid, benzenesulphonic acid, naphthalenedisulphonic acid, acetic acid, trifluoroacetic acid, propionic acid, lactic acid, tartaric acid, malic acid, citric acid, fumaric acid, maleic acid and benzoic acid.

Physiologically acceptable salts of lysobactin also include salts of conventional bases such as, by way of example and preferably, alkali metal salts (e.g. sodium and potassium salts), alkaline earth metal salts (e.g. calcium and magnesium salts) and ammonium salts derived from ammonia or organic amines having 1 to 16 C atoms, such as, by way of example and preferably, ethylamine, diethylamine, triethylamine, ethyldiisopropylamine, monoethanolamine, diethanolamine, triethanolamine, dicyclohexylamine, dimethylaminoethanol, procaine, dibenzylamine, N-methylmorpholine, arginine, lysine, ethylenediamine and N-methylpiperidine.

In the context of the present invention, the term "aminoglycoside antibiotic" or "aminoglycoside", refers to oligosaccharide substances that are composed of cyclohexane-sugar-groups and amino-sugar-groups and that functionally share the feature of attaching to the 30S subunit of membrane associated ribosomes to thereby interfere with protein biosynthesis of (particularly) bacterial cells. Non-limiting and non-exhaustive members of the group of aminoglycosides pursuant to the present invention are those selected from the list consisting of Amikacin, Apramycin, Framycetin, Geneticin (G418), Gentamicin, Kanamycin, Netilmicin, Neomycin, Paromomycin, Sisomycin, Spectinomycin, Streptomycin and Tobramycin.

As with lysobactin, "aminoglycoside antibiotic" or "aminoglycoside" also encompasses physiologically acceptable salts thereof. Therefore physiologically acceptable salts of aminoglycosides include acid addition salts of mineral acids, carboxylic acids and sulphonic acids as well as salts of conventional bases as listed with lysobactin in the context of the present invention.

The lysobactin and aminoglycosides can act systemically and/or locally. For this purpose, it can be administered in a suitable way such as, for example, by the oral, parenteral, pulmonary, nasal, sublingual, lingual, buccal, rectal, intraauricular, dermal or transdermal route, or as implant or stent.

The lysobactin and aminoglycosides can be administered in administration forms suitable for these administration routes.

Suitable for oral administration are administration forms which function according to the prior art and deliver lysobactin and aminoglycosides rapidly and/or in modified fashion, and which contain lysobactin and aminoglycosides in crystalline and/or amorphized and/or dissolved form, such as, for example, tablets (uncoated or coated tablets, for example having enteric coatings or coatings which are insoluble or dissolve with a delay and control the release of lysobactin), tablets which disintegrate rapidly in the mouth, or films/wafers, films/lyophilizates, capsules (for example hard or soft gelatin capsules), sugar-coated tablets, granules, pellets, powders, emulsions, suspensions, aerosols or solutions.

Parenteral administration can take place with avoidance of an absorption step (e.g. intramammary, intravenous, intraarterial, intracardiac, intraspinal or intralumbar) or with inclusion of absorption (e.g. intramuscular, subcutaneous, intracutaneous, percutaneous or intraperitoneal). Administration forms suitable for parenteral administration are, inter alia, preparations for injection and infusion in the form of solutions, suspensions, emulsions, lyophilizates or sterile powders.

Suitable for the other administration routes are, for example, pharmaceutical forms for aqueous suspensions (lotions, shaking mixtures), lipophilic suspensions, ointments, creams, transdermal therapeutic systems (such as, for example, patches), milk, pastes, foams, dusting powders, implants or stents.

Lysobactin and aminoglycosides can be converted into the stated administration forms. This can take place in a manner known per se by mixing with inert, nontoxic, pharmaceutically suitable excipients. These excipients include, inter alia, carriers (for example microcrystalline cellulose, lactose, mannitol), solvents (e.g. liquid polyethylene glycols), emulsifiers and dispersants or wetting agents (for example sodium dodecyl sulphate, polyoxysorbitan oleate), binders (for example polyvinylpyrrolidone), synthetic and natural polymers (for example albumin), stabilizers (e.g. antioxidants such as, for example, ascorbic acid), colours (e.g. inorganic pigments such as, for example, iron oxides) and masking flavours and/or odours.

The present invention will be further described with reference to the following aspects and embodiments. They may be combined freely unless the context clearly indicates otherwise.

One embodiment concerns the combination of lysobactin with at least one aminoglycoside antibiotic for use in the treatment of mastitis, wherein lysobactin and the at least one aminoglycoside antibiotic are administered intramammarily. Preferably the aminoglycoside antibiotic is neomycin or kanamycin. More preferably the mastitis is bovine mastitis.

Another embodiment concerns the combination of lysobactin with at least one aminoglycoside antibiotic for use in the treatment of mastitis, wherein the mastitis is a clinically manifest mastitis. Preferably the aminoglycoside antibiotic is neomycin or kanamycin. More preferably the mastitis is bovine mastitis.

Another embodiment concerns the combination of lysobactin with at least one aminoglycoside antibiotic for use in the treatment of mastitis, wherein the mastitis is a subclinical bovine mastitis. Preferably the aminoglycoside antibiotic is neomycin or kanamycin. More preferably the mastitis is bovine mastitis.

Another embodiment concerns the combination of lysobactin with at least one aminoglycoside antibiotic for use in the treatment of mastitis, wherein the lysobactin is provided at a dose of 25 to 1000 mg per udder quarter, preferably at a dose of 50 to 500 mg per udder quarter and more preferably at a dose of 100 to 300 mg per udder quarter. Most preferably the mastitis is bovine mastitis.

In the preferred embodiment of this embodiment the combination of lysobaction is made with neomycin or kanamycin, with a preference for neomycin.

It may also be necessary where appropriate to deviate from the stated amounts; in particular as a function of the bodyweight, route of administration, individual response to the active ingredient, nature of the preparation and time or interval over which administration takes place. Thus, it may be sufficient in some cases to make do with less than the aforementioned minimum amount, whereas in other cases the stated upper limit must be exceeded. It may in the event of administration of larger amounts be advisable to divide these into a plurality of individual doses over the day.

Depending on the actual aminoglycoside antibiotic actually used pursuant to the present invention, the above referred to doses thereof may vary and thus the ratio of lysobactin in relation thereto.

Very positive results on both Gram-negative as well as Gram-positive pathogens are achieved and it is therefore preferred if the ratio of lysobactin to aminoglycoside antibiotic is at least 0.1. The ratio of at least 0.2 provides with even better results, while it is more preferred to have a ratio of at least 1:1 and even more preferred is a ratio of at least 2:1. Most preferred is a ratio of at least 4:1.

Another embodiment concerns the combination of lysobactin with at least one aminoglycoside antibiotic for use in the treatment of mastitis, wherein the mastitis is caused by Gram-positive and Gram-negative bacteria. Preferably the mastitis is bovine mastitis.

More preferably, this concerns the combination of lysobactin with at least one aminoglycoside antibiotic for use in the treatment of bovine mastitis, wherein the bovine mastitis is caused by *Staphylococcus* bacteria, *Streptococcus* bacteria, *Trueperella* bacteria, *Corynebacterium* bacteria and/or *Escherichia* bacteria.

In particular, the bovine mastitis may be caused by *Staphylococcus aureus,* coagulase-negative staphylococci, *Streptococcus uberis, Streptococcus dysgalacticae Streptococcus agalacticae* and/or *E. coli* bacteria.

Furthermore, the bovine mastitis may be caused by *Trueperella pyogenes* and/or E. *coli.* Also, the bovine mastitis may be caused by *Corynebacterium bovis* and/or *E. coli.*

*Staphylococcus aureus,* coagulase-negative staphylococci, *Streptococcus uberis, Streptococcus dysgalacticae Streptococcus agalacticae, Trueperella pyogenes,* and *Corynebacterium bovis* are the most common pathogens found in cases of bovine mastitis, while superinfection with *Escherichia* is as common and they were collectively found to be particularly susceptible to a treatment with a combination of lysobactin with at least one aminoglycoside antibiotic according to the present invention. A mastits can also become clinically relevant solely by presence of *Escherichia* bacteria Another aspect of the present invention is a pharmaceutical composition formulated for intramammary administration into mammaries, wherein the composition comprises lysobactin at least one aminoglycoside antibiotic. Preferably the composition is formulated to be administered into bovine mammaries. More preferably, the composition further comprises a pharmaceutically acceptable excipient and in particular a pharmaceutically acceptable carrier.

These excipients include, inter alia, carriers (for example microcrystalline cellulose, lactose, mannitol), solvents (e.g. liquid polyethylene glycols), emulsifiers and dispersants or wetting agents (for example sodium dodecyl sulphate, polyoxysorbitan oleate), binders (for example polyvinylpyrrolidone), synthetic and natural polymers (for example albumin), stabilizers (e.g. antioxidants such as, for example, ascorbic acid), colours (e.g. inorganic pigments such as, for example, iron oxides) and masking flavours and/or odours.

Also described herein is the use of lysobactin and at least one aminoglycoside antibiotic for the preparation of pharmaceuticals for the treatment of mastitis. Use for the preparation of pharmaceuticals for the treatment of bovine mastitis is also described herein.

In the use described herein the bovine mastitis is a clinically manifest bovine mastitis.

In the use described herein the bovine mastitis is a subclinical bovine mastitis.

In use described herein the bovine mastitis is caused by *Staphylococcus* bacteria, *Streptococcus* bacteria, *Trueperella* bacteria, *Corynebacterium* bacteria, and/or *Escherichia* bacteria.

In particular, the bovine mastitis may be caused by *Staphylococcus aureus,* coagulase-negative staphylococci, *Streptococcus uberis, Streptococcus dysgalacticae, Streptococcus agalacticae,* and/or *E. coli* bacteria.

Furthermore, the bovine mastitis may be caused by *Trueperella pyogenes* and/or *E.coli.* Also, the bovine mastitis may be caused by *Corynebacterium bovis* and/or *E.coli.*

Further described herein is a method of treating mastitis, the method comprising the step of administering to a female non-human animal having an udder and being in need thereof a therapeutically effective amount of lysobactin and at least one aminoglycoside antibiotic. As described herein the female non-human animal having an udder is a cow.

Finally, even though major aspects of the present invention are directed to the treatment of - particularly bovine - mastitis for commercial applicability reasons, the synergy surprisingly found herein cannot be found to be restricted to that specific treatment of an - particularly bovine - udder. The experimental data provided herein show that the above referred to synergy applies to any treatment of non-human animals suffering from any disease caused by Gram-negative and Gram-positive bacteria.

Therefore the overarching embodiment of the present invention is generally concerned with the combination of lysobactin and at least one at least one aminoglycoside antibiotic for use in a treatment of a non-human mammalian animal against a disease caused by Gram-negative and Gram-positive bacteria.

### Examples:

The present invention will be further elucidated with reference to the following examples and figures without being limited to them.
FIG. 1 shows the kill kinetics of lysobactin against *Staphylococcus aureus* and *Streptococcus uberis* (example 2)
FIG.2 shows the MIC changes of lysobactin against *Staphylococcus aureus* and *Streptococcus uberis* during serial passaging (example 4)
FIG. 3 shows the efficacy of lysobactin in a *Staphylococcus aureus* acute mouse mastitis model (example 5)
FIG. 4 shows the efficacy of lysobactin in a *Streptococcus uberis* challenge mouse mastitis model (example 6)
FIG. 5 shows the concentration-time profile of lysobactin in milk after intramammary (IMAM) application to lactating Holstein cows (example 7)
FIG. 6 shows the kill kinetics in milk of lysobactin and neomycin, either alone or in combination at 1:1 and 2:1 dose ratios, against *Staphylococcus aureus* and *Streptococcus uberis* (example 10)
FIG. 7 shows the kill kinetics in milk of lysobactin and neomycin, either alone or in combination at 1:5 and 1:10 dose ratios, against *Staphylococcus aureus* and *Streptococcus uberis* (example 10)
FIG. 8 shows the kill kinetics in milk of lysobactin and neomycin, either alone or in combination, against E. *coli* (example 10)
FIG. 9 shows the kill kinetics of combinations of lysobactin, either with neomycin or kanamycin, against *Staphylococcus aureus* (example 11)
FIG. 10 shows the kill kinetics of combinations of lysobactin with florfenicol for *Staphylococcus aureus* and *Streptococcus uberis* (example 12)
FIG. 11 shows the efficacy of lysobactin and neomycin, either in combination or alone, in a *Staphylococcus aureus* acute mouse mastitis model (example 13)
FIG. 12 shows the efficacy of lysobactin and neomycin, either in combination or alone, in a *Streptococcus uberis* acute mouse mastitis model (example 14)

### Example 1: In vitro antibacterial activity of lysobactin alone against mastitis pathogens

The in vitro antibacterial activity of lysobactin against common mastitis pathogens such as *Staphylococcus aureus,* Coagulase-negative staphylococci, *Streptococcus uberis, Streptococcus dysgalactiae, Streptococcus agalactiae, Trueperella pyogenes, Escherichia coli,* or *Klebsiella pneumoniae* was assessed by microbroth dilution MIC methodology as described by the Clinical and Laboratory Standards Institute (CLSI) in order to obtain the Minimal Inhibitory Concentration (MIC), which is defined as the lowest concentration of a substance that prevents visible growth of a bacterium. The results expressed as MIC₉₀ are summarized in the following table, where the MIC₉₀ is defined as the concentration at which the growth of at least 90% of the strains of a given species is inhibited.

| **Tested bacteria** | **MIC₉₀ [µg/ml\| of lysobactin** |
|---|---|
| *Staphylococcus aureus* | 0.5 |
| Coagulase-negative staphylococci | 0.5 |
| *Streptococcus uberis* | 0.125 |
| *Streptococcus dysgalactiae* | 0.25 |
| *Streptococcus agalactiae* | 0.25 |
| *Trueperella pyogenes* | 0.5 |
| *Escherichia coli* | >16 |
| *Klebsiella pneumoniae* | >16 |

### Example 2: In vitro kill kinetics of lysobactin alone for mastitis pathogens

In order to assess the ability of lysobactin to kill bacteria in milk, flasks containing different concentrations of lysobactin in store-bought full-fat milk were inoculated with 1-2 x 10⁶ colony forming units/ml of a representative strain of either *Staphyloccus aureus* or *Streptococcus uberis.* The flasks were incubated for 24-48 hours in a shaking water bath at 35 +/- 2°C, and viable bacterial counts in each flask were determined at several time-points by diluting and plating samples on agar plates. A reduction of the number of viable bacteria in the initial inoculum by at least 99.9% is defined as bactericidal activity.

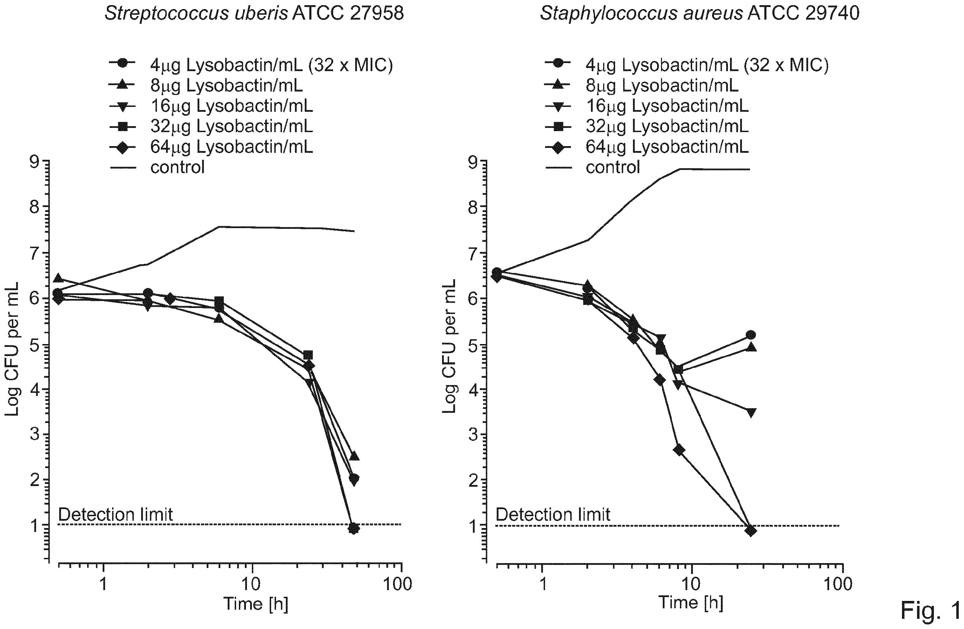

The kill kinetics of lysobactin against *Staphylococcus aureus* ATCC 29740 and *Streptococcus uberis* ATCC 27958 in milk were determined for concentrations of lysobactin of 4, 8, 16, 32 and 64 µg/mL. Results are depicted in FIG. 1 (CFU: colony forming units). A cidality at 24 h and 48 h, respectively, can be postulated for *S. aureus* and *S. uberis.*

### Example 3: In vitro assessment of spontaneous resistance development against lysobactin alone:

The frequency of spontaneous resistance development was assessed by plating at least 1x10⁹ colony forming units of the respective bacterial strain on agar plates containing lysobactin at either 4x or 8x the MIC and incubating the plates at 35 +/- 2°C. After 48 h, the number of colonies that had grown on the plates at lysobactin concentration above the MIC was divided by the number of bacteria that was initially plated. The resulting number is defined as the spontaneous resistance frequency, and is an indication for the likelihood of resistant isolates to appear during an infection.

As a conclusion, lysobactin at 4- and 8-fold MIC displays a very good resistance profile: no resistant isolates were detectable. The results are summarized in the following tables:
*Staphylococcus aureus* ATCC 29740:

| **Lysobactin concentration** | **plated CFU** | **24 h** | | **48 h** | |
|---|---|---|---|---|---|
| | | **obtained colonies** | **frequency of resistance** | **obtained colonies** | **frequency of resistance** |
| 2 µg/ml (4x MIC) | 3.52 x 10⁹ | 0 | < 2.84 x 10⁻¹⁰ | 0 | < 2.84 x 10⁻¹⁰ |
| 4 µg/ml (8x MIC) | 3.52 x 10⁹ | 0 | < 2.84 x 10⁻¹⁰ | 0 | < 2.84 x 10⁻¹⁰ |

*Streptococcus uberis* ATCC 27958:

| **Lysobactin concentration** | **plated CFU** | **24 h** | | **48 h** | |
|---|---|---|---|---|---|
| | | **obtained colonies** | **frequency of resistance** | **obtained colonies** | **frequency of resistance** |
| 2 µg/ml (4x MIC) | 2.4 x 10¹⁰ | 0 | < 4.17 x 10⁻¹¹ | 0 | < 4.17 x 10⁻¹¹ |
| 4 µg/ml(8x MIC) | 2.4 x 10¹⁰ | 0 | < 4.17 x 10⁻¹¹ | 0 | < 4.17 x 10⁻¹¹ |

### Example 4: In vitro assessment of resistance development against lysobactin alone during serial passaging

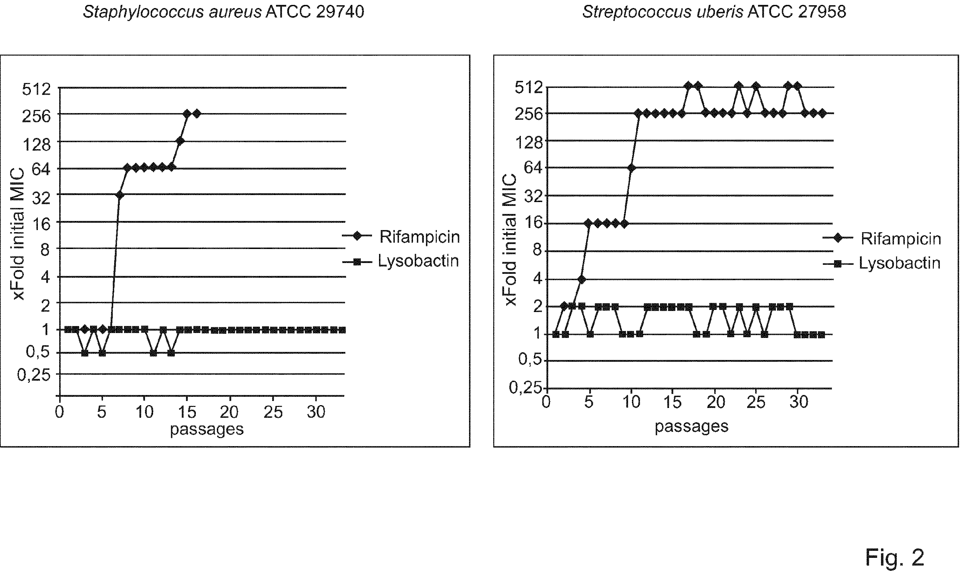

The appearance of MIC changes during constant exposure of bacteria to sub-MIC concentrations of lysobactin was assessed by serial passaging experiments. On the first day the MICs of S. *aureus* and *S. uberis* were assessed by microbroth dilution MIC methodology as described by the Clinical and Laboratory Standards Institute (CLSI). Each day for the following 33 consecutive days, bacteria from the well containing the highest concentrations allowing full growth were collected and used to inoculate new 96-well plates and to assess the MIC during constant exposure to lysobactin. The MIC obtained on each day was plotted over time. The results are shown in FIG. 2. Rifampicin, which is known to result in quick changes of the MIC, was used as positive control.

The results indicate a very good profile of lysobactin for resistance development during constant exposure, as the MICs for *S. aureus* and *S. uberis* remain constant over the 33 days period.

### Example 5: Efficacy of lysobactin alone in an acute mouse mastitis model with S. aureus

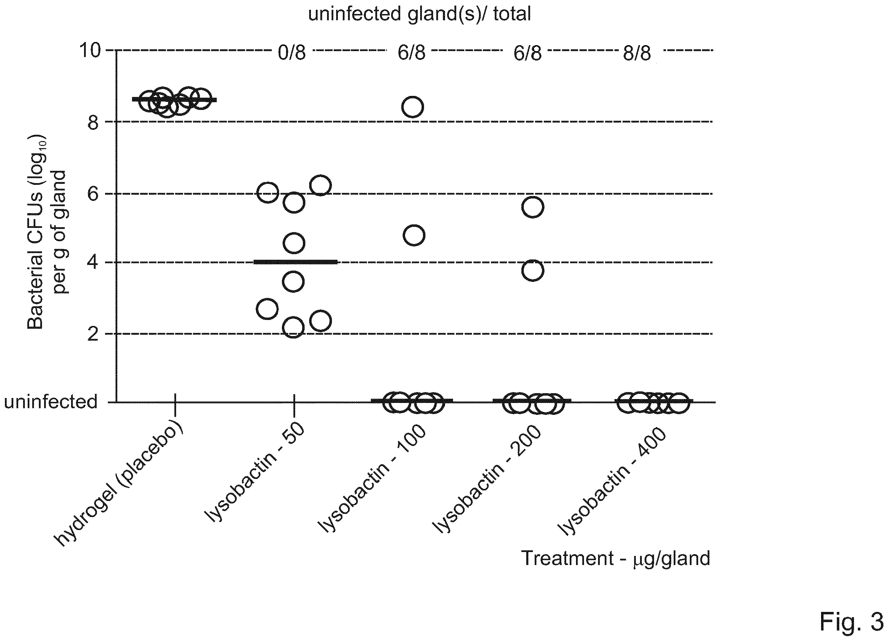

The efficacy of lysobactin (formulated in a hydrogel at pH 4.7) was tested in a *Staphylococcus aureus* acute mouse mastitis model established at the University of Sherbrooke, Canada (Brouillette et al, Vet. Microbiol. (2004) 4:253-262). Both the abdominal mammary glands (L4 and R4) of lactating CD-1 mice were intramammarily infected with 100 CFU (colony forming units) of *S. aureus.* The mice were treated intramammarily with lysobactin four hours after infection. Each treatment group contained at least 3 mice (6 quarters), 14 hours later (18 hours after inoculation) mice were sacrificed, mammary glands were harvested and the CFU content evaluated by plating 10-fold serial dilutions of mammary gland homogenates. The CFU content was expressed as log₁₀ count. The detection limit was 200 CFU/g of gland. Glands with less than 200 CFU/g were regarded as cleared. The results are shown in FIG. 3.

Intramammary instillation of 50 µg lysobactin reduces the median CFU content by ca. 4 log₁₀, 400 µg lysobactin eliminates the infection from all infected glands.

### Example 6: Efficacy of lysobactin alone in an acute mouse mastitis model with S. uberis

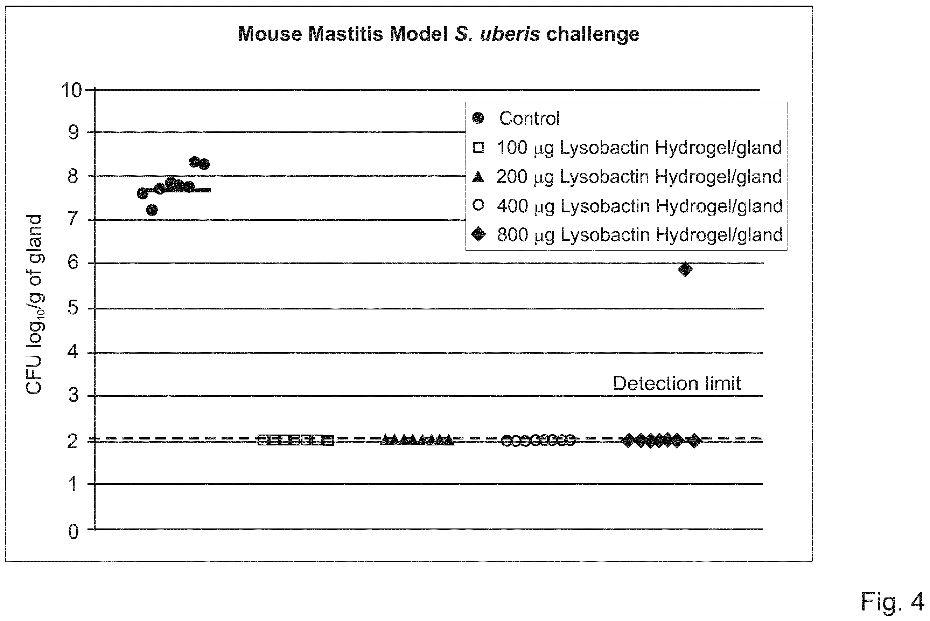

The efficacy of lysobactin (formulated in a hydrogel at pH 4.7) was tested in a *Streptococcus uberis* challenge mouse mastitis model. The results are shown in FIG. 4.

Twenty lactating mice were experimentally infected on the fourth pair of mammary glands with S. *uberis* around 12-15 days after birth of the offspring. Four hours after inoculation, groups of four mice were treated on the same glands with lysobactin at 100, 200, 400, or 800 µg/ gland formulated as hydrogel. The fifth group was treated solely with the hydrogel vehicle as negative control. Eighteen hours after infection the animals were euthanized, the glands were removed, homogenized, and bacterial colony forming units (CFUs) were determined by established microbiological methods. Subsequently CFU/mL homogenate as well as CFU/g of gland were calculated. The detection limit was approximately 100 CFU/g of gland. Antimicrobial activity of lysobactin against *Strep uberis* was determined by comparison of the mean CFUs/gland of the different dosing groups and the negative control group.

Glands of all animals of the control group showed an optimum infection rate (≥10⁷ CFU/g of gland) 18 hours after infection. With one exception in the highest dosing group all glands in the lysobactin treated groups had bacterial counts below the limit of detection (10² CFU/g). It can be concluded that lysobactin formulated as hydrogel has outstanding antibacterial efficacy against *S. uberis* at intramammary doses between 100 and 800 µg/gland.

### Example 7: Lysobactin alone in vivo cattle data - milk pharmacokinetic study in lactating Holstein cows

The study was designed as a non-pivotal study suitable to investigate the milk pharmacokinetics of the active substance lysobactin after single intracisternal application to lactating dairy cows.

The active substance was provided in a paraffine based service formulation suitable for intracisternal application containing 150 mg lysobactin in 10 g oily suspension.

The test item was administered as single intracisternal treatment at a dose rate of 150 mg lysobactin to a single hind quarter of four lactating dairy cows each.

The dairy cows on study (Holstein cows) represented the target population in age, lactation number, lactation stage, milk yield and breed. The animals were stalled in a tie-barn and were fed with standard feed for dairy cows consisting of corn and gras silage and milk performance feed. Milking was twice daily at a 12 hour interval using a bucket-type milking device.

Frequent milk sampling was performed from the treated and respective control quarters prior to (0 h) and over a period of 168 h after treatment (0.5, 1, 2, 4, 6, 8, 12, 24, 36, 48, 60, 72, 84, 96, 120, 144, and 168 h) by manual stripping of the respective udder quarters. Milk samples at routine milking times were gathered prior to milking.

Concentrations of the active substance lysobactin in milk were analyzed by HPLC with detection by tandem mass spectrometry. The limit of quantitation was 0.05 mg/L.

Pharmacokinetic evaluation of milk concentration data was based on non-compartmental methods and comprised PK-parameters to adequately describe the absorption, distribution and elimination profile of the active substance in milk.

Summarized results derived from the treated udder quarters are presented in the following table. No lysobactin was detected in the control samples (untreated udder quarters).

### Mean milk pharmacokinetic results of lysobactin after single treatment

| Matrix | Cₘₐₓ¹ mg/L | Tₘₐₓ² h | t_{1/2}¹ h | AUC_{inf}¹ mg^{∗}h/L | AUC₀₋₁₂ₕ¹ mg^{∗}h/L | AUC₀₋₂₄ₕ¹ mg^{∗}h/L |
|---|---|---|---|---|---|---|
| Milk | 342 | 4 | 11.6 | 2321 | 1870 | 2213 |

| | | | | | | |
|---|---|---|---|---|---|---|
| *Dose applied to 1 quarter per cow was 150 mg lysobactin;* *Means are given as 1) geometric mean, 2) median* | | | | | | |

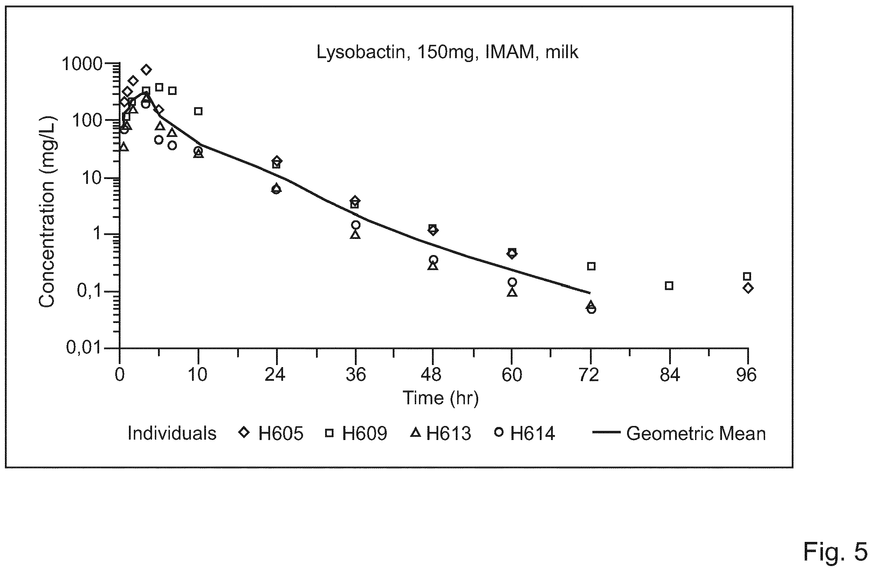

The concentration time curve of lysobactin is depicted in FIG. 5.

### Example 8: Lysobactin alone in vivo cattle data - dairy cow udder infection model with S. aureus

Fifteen healthy lactating dairy cows were experimentally infected with the mastitis pathogen *Staphylococcus aureus* on all four udder quarters. As soon as an udder quarter showed clinical symptoms of mastitis, such as swelling, pain, abnormal milk consistency, it was treated with either Lysobactin paraffine based ointment at two different concentrations, or Ubrolexin® (Cephalexin + Kanamycin, Boehringer Ingelheim), or saline solution as negative control. Treatments were randomly assigned to 42 udder quarters in total, either 50 mg lysobactin per quarter (in 11 quarters), or 150 mg Lysobactin (in 11 quarters), or Ubrolexin® (in 9 quarters), or saline solution (in 11 quarters). Only udder quarters that were bacteriologically positive for the challenge organism immediately prior to treatment (n=42) were eligible for assessment of microbiological and clinical cure. The diseased quarters were treated with these intramammary formulations two times with an interval of 24 hours in between. Udders were clinically examined and milk samples were taken before treatment and several times after that until three weeks after the second administration. Milk was inspected visually for deviation in its consistency and samples were evaluated for presence of the challenge organism and for somatic cell count to confirm the diagnosis mastitis. Diseased udder quarters were considered microbiologically cured when *Staphylococcus aureus* found in milk samples shortly before treatment could not be isolated from any milk sample taken within the time period between the third and the twenty-first day after the second treatment. Clinical cure was achieved when the local symptoms of mastitis had completely disappeared, and recovery of somatic cell counts (SCC) as parameter of udder inflammation was attained when all counts remained below 500.000 cells per mL of milk in the period mentioned above.

The microbiological cure rate on Day 3 and Day 21 after second treatment was 73% and 27% for Lysobactin 50 mg, 73% and 45% for Lysobactin 150 mg, 78% and 44% for Ubrolexin®, and 0% and 0% for saline solution, respectively. The local clinical cure rate on Day 3 and Day 21 after second treatment was 45% and 73% for Lysobactin 50 mg, 37% and 82% for Lysobactin 150 mg, 33% and 67% for Ubrolexin®, and 55% and 73% for saline solution, respectively. The SCC recovery rate on Day 3 and Day 21 after second treatment was 36% and 45% for Lysobactin 50 mg, 36% and 91% for Lysobactin 150 mg, 56% and 67% for Ubrolexin®, and 36% and 27% for saline solution, respectively.

It can be concluded that Lysobactin 150 mg shows similar or even better efficacy in comparison to the positive control product Ubrolexin® and superiority to Lysobactin 50 mg and Saline solution in the parameters microbiological and clinical cure, and SCC recovery.

### Example 9: Lysobactin alone in vivo cattle data - dairy cow udder infection model with S. uberis

Seventy-two healthy lactating dairy cows were experimentally infected with the mastitis pathogen *Streptococcus uberis* on two udder quarters per cow. As soon as an udder quarter showed clinical symptoms of mastitis (e.g. heat, swelling, redness, pain, abnormal milk consistency), it was treated with either lysobactin paraffine based ointment at two different concentrations, or Ubrolexin® (cephalexin + kanamycin, Boehringer Ingelheim).

Treatments were randomly assigned to 41 (clinical) or 37 (microbiological) udder quarters in total, either 50 mg lysobactin per quarter (in 15/13 quarters), or 150 mg lysobactin (in 15/14 quarters), or Ubrolexin® (in 11/10 quarters) as positive control. The diseased quarters were treated with these intramammary formulations two times with an interval of 24 hours in between. Udders were clinically examined and milk samples were taken before treatment and several times after that until three weeks after the second administration. Milk was inspected visually for deviation in its consistency and samples were evaluated for presence of the challenge organism to confirm the diagnosis mastitis. Diseased udder quarters were considered microbiologically cured when *Streptococcus uberis* found in milk samples shortly before treatment could not be isolated from any milk sample taken within the time period between the seventh and the twenty-first day after the second treatment. Clinical cure was achieved when the local symptoms of mastitis completely disappeared or were at the most of very slight nature.

The microbiological cure rate on Day 7 and Day 21 after second treatment was 84.6% and 92.31% for lysobactin 50 mg, 71.4% and 85.71% for lysobactin 150 mg, and 30% and 30% for Ubrolexin®, respectively. The local clinical cure rate on Day 7 after second treatment was 86.7% both for lysobactin 50 mg and lysobactin 150 mg, and 36.4% for Ubrolexin®, respectively.

It can be concluded that lysobactin 50 and 150 mg were clearly more efficacious in comparison to the positive control product Ubrolexin® in the pivotal parameters microbiological and clinical cure.

### Example 10: In vitro kill kinetics of combinations of lysobactin with neomycin for mastitis pathogens

In order to assess the synergistic activity of lysobactin and neomycin in store-bought full-fat milk *in vitro*, flasks containing different concentrations of lysobactin and neomycin, either alone or in combination, were inoculated with 1-2 x 10⁶ colony forming units/ml of a representative strain of either *Staphyloccus aureus, Streptococcus uberis* or *E. coli.*

The flasks were incubated for 24-48 hours in a shaking water bath at 35 +/- 2°C, and viable bacterial counts in each flask were determined at several time-points by diluting and plating samples on agar plates.

A reduction of the number of viable bacteria achieved by the combination compared to the most active single compound by at least 99% after 24 h is defined as synergistic activity (NCCLS/CLSI M26-A Vol. 19, No.18).

The kill kinetics of lysobactin and neomycin, either alone or in combination, against *Staphylococcus aureus* ATCC 29740 in milk were determined for concentration ratios of lysobactin:neomycin of 1:1 (2 µg/ml each) and 2:1 (2 µg/ml of lysobactin and 1 µg/ml neomycin).

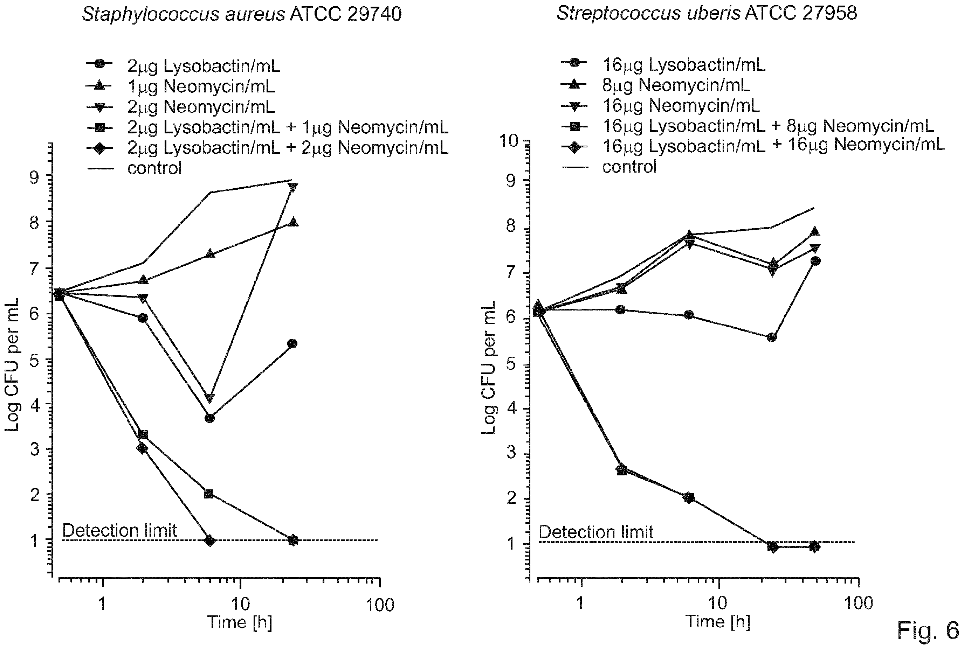

Results are depicted in FIG. 6.

After 24 h a synergistic activity of the lysobactin:neomycin combinations could be observed. The synergy is extremely visible at ratios of at least 2:1, particularly at a ratio of 1:1.

The kill kinetics of lysobactin and neomycin, either alone or in combination, against *Streptococcus uberis* ATCC 27958 in milk were determined for concentration ratios of lysobactin:neomycin of 1:1 (16 µg/ml each) and 2:1 (16 µg/ml of lysobactin and 8 µg/ml neomycin).

Results are also depicted in FIG. 6.

After 24 h a synergistic activity of the lysobactin:neomycin combinations could be observed at both ratios.

The kill kinetics of lysobactin and neomycin, either alone or in combination, against *Staphylococcus aureus* ATCC 29740 in milk were determined for concentration ratios of lysobactin:neomycin of 1:5 (1 µg/ml lysobactin plus 5 µg/ml neomycin and 1.5 µg/ml lysobactin plus 7.5 µg/ml neomycin, respectively).

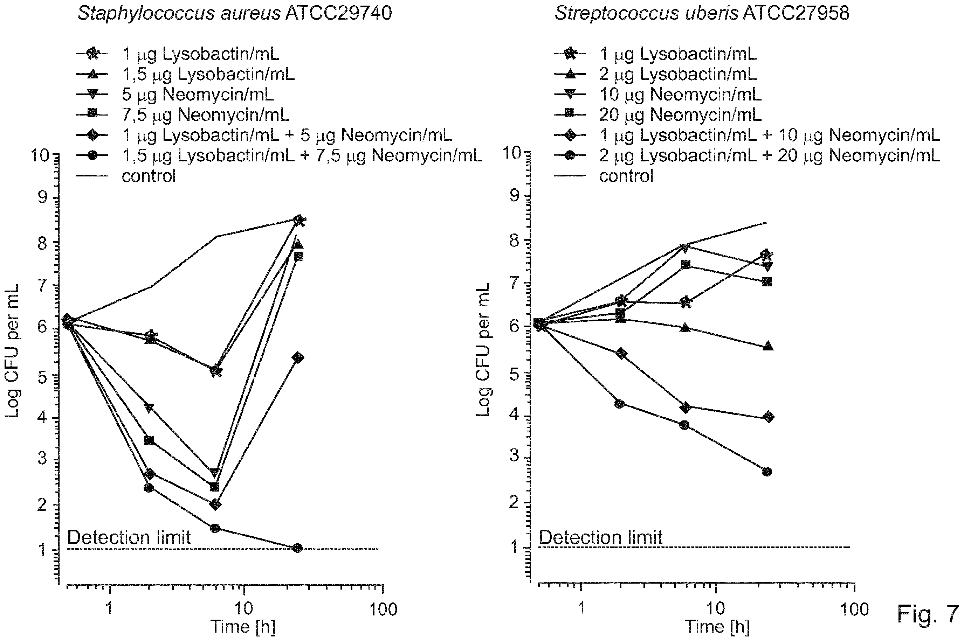

Results are depicted in FIG. 7.

After 24 h a synergistic activity could be observed. The synergy is extremely visible already at lysobactin:neomycin ratios of at least 1:5 (i.e. 0.2). Said synergy is not dependent on the absolute amount of lysobactin and neomycin used, as evidenced by FIG. 7.

The kill kinetics of lysobactin and neomycin, either alone or in combination, against *Streptococcus uberis* ATCC 27958 in milk were determined for concentration ratios of lysobactin:neomycin of 1:10 (1 µg/ml lysobactin plus 10 µg/ml neomycin and 2 µg/ml lysobactin plus 20 µg/ml neomycin, respectively).

Results are also depicted in FIG. 7.

After 24 h a synergistic activity could be observed. At a lysobactin:neomycin ratio of 1:10 (0.1) the synergy is highly visible regardless of absolute amount of lysobactin and neomycin.

Taken together, a synergistic activity of lysobactin:neomycin combination could be shown. Said synergistic activity being very visible at ratios ranging from 2:1 to 1:5 (0.2) and 2:1 to 1:10 (0.1) for *Staphylococcus aureus* ATCC 29740 and *Streptococcus uberis* ATCC 27958, respectively.

The kill kinetics of lysobactin and neomycin, either alone or in combination, against *E. coli* P4:O32 in milk were determined for concentration ratio of lysobactin:neomycin of 4:1 (256 µg/ml lysobactin and 32 µg/ml neomycin).

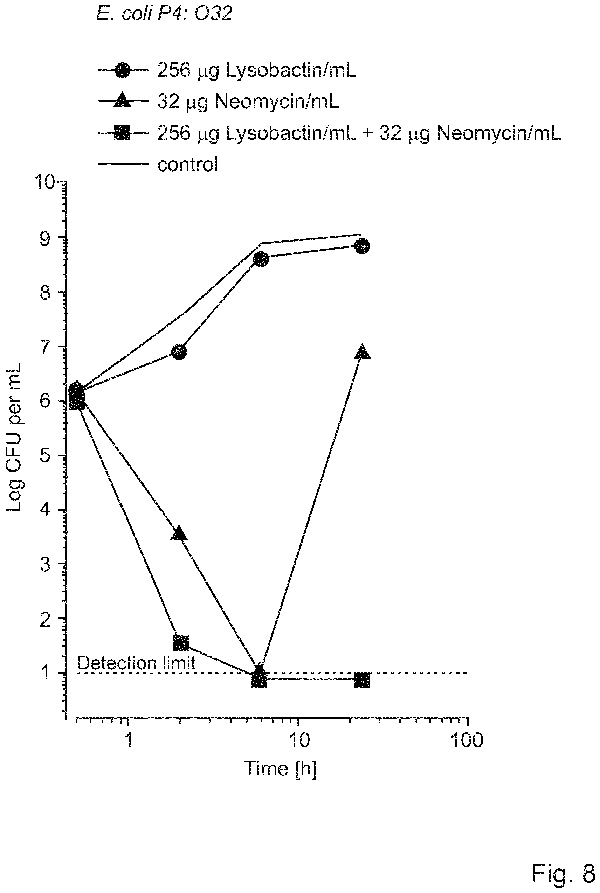

Results are depicted in FIG. 8.

Although neomycin as well as the lysobactin:neomycin combination were able to reduce the bacterial cell count below the detection limit after 8 h, the combination, showed a faster onset of killing.

Furthermore the combination substantially prevented regrowth of the bacteria after 24h which cannot be achieved by the respective sole actives.

Therefore, a synergistic activity was observed here as well with regard to the Gram-negative *E. coli* bacteria.

### Example 11: In vitro kill kinetics of combinations of lysobactin either with neomycin or kanamycin for Staphylococcus aureus ATCC 29740

To investigate whether the above referred to synergy does apply to more aminoglycoside antibiotics beyond neomycin combined with lysobactin, the kill kinetics of kanamycin as combination partner were tested as well.

Kill kinetics were performed as described in example 10.

The kill kinetics of lysobactin either with neomycin or kanamycin against *Staphylococcus aureus* ATCC 29740 in milk were determined for a concentration ratio of lysobactin:neomycin or lysobactin:kanamycin of 1:4 (0.5 µg/ml lysobactin plus 2 µg/ml neomycin or kanamycin; 1 µg/ml lysobactin plus 4 µg/ml neomycin or kanamycin and 2 µg/ml lysobactin plus 8 µg/ml neomycin or kanamycin, respectively).

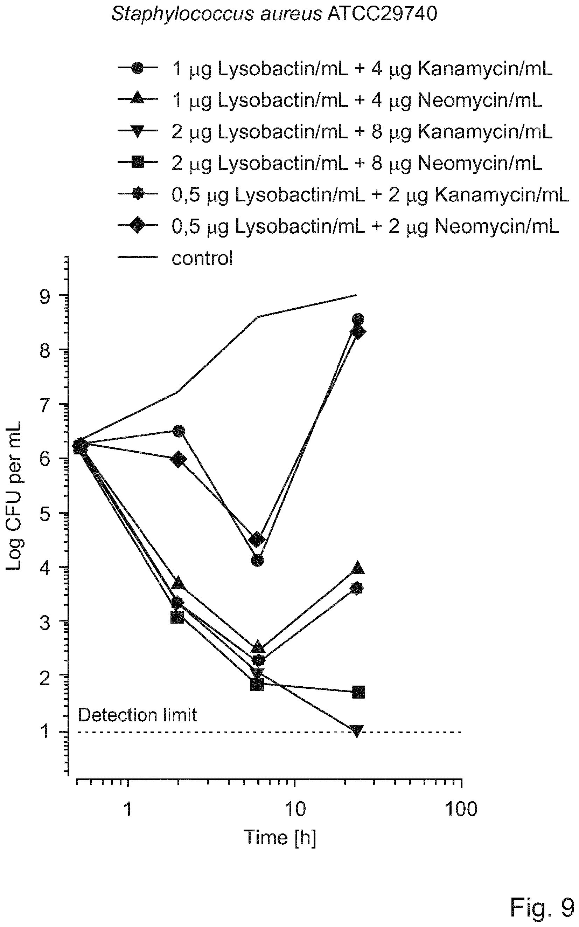

Results are depicted in FIG. 9.

For all concentrations evaluated the lysobactin:kanamycin combination showed similar activity compared to the lysobactin:neomycin combination.

From that it can be concluded that the combination of lysobactin with aminoglycoside antibiotics *per* se conveys an unexpected synergy.

### Comparative Example 12: In vitro kill kinetics of combinations of lysobactin with florfenicol for Staphylococcus aureus ATCC 29740 and Streptococcus uberis ATCC 27958

Synergistic activities of two different antibiotic agents are not reliably predictable, as shown hereafter.

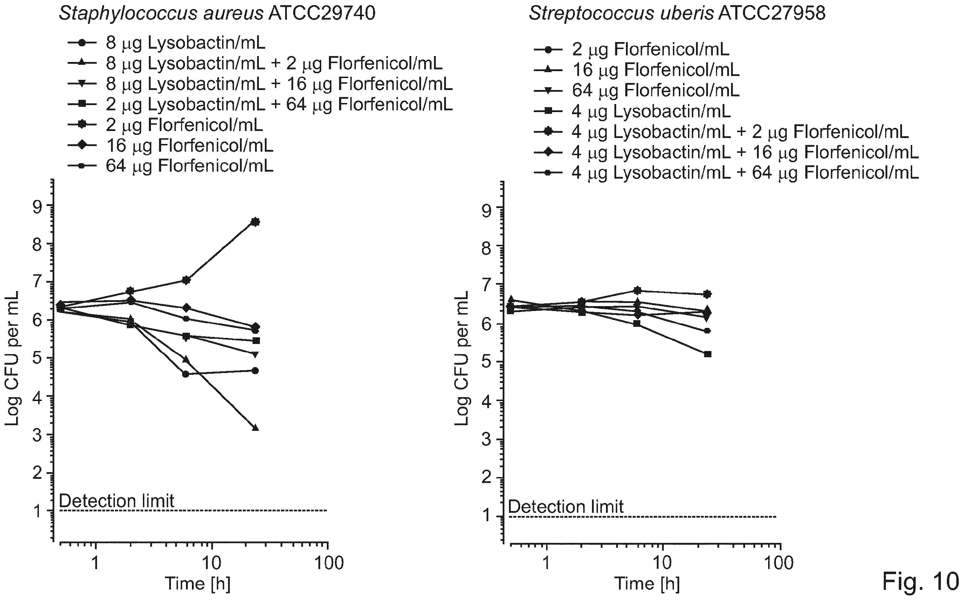

For example, the combination of lysobactin with florfenicol did not reveal synergistic activity for neither *Staphylococcus aureus* ATCC 29740 nor *Streptococcus uberis* ATCC 27958 in milk under test conditions depicted in FIG. 10.

### Example 13: Efficacy of a combination of lysobactin with neomycin trisulfate in an acute mouse mastitis model with S. aureus

The efficacy of lysobactin and neomycin trisulfate, either alone or in combination, was tested in a *Staphylococcus aureus* acute mouse mastitis model established at the University of Sherbrooke, Canada (Brouillette et al, Vet. Microbiol. (2004) 4:253-262), as described in example 5. Samples were formulated in paraffin.

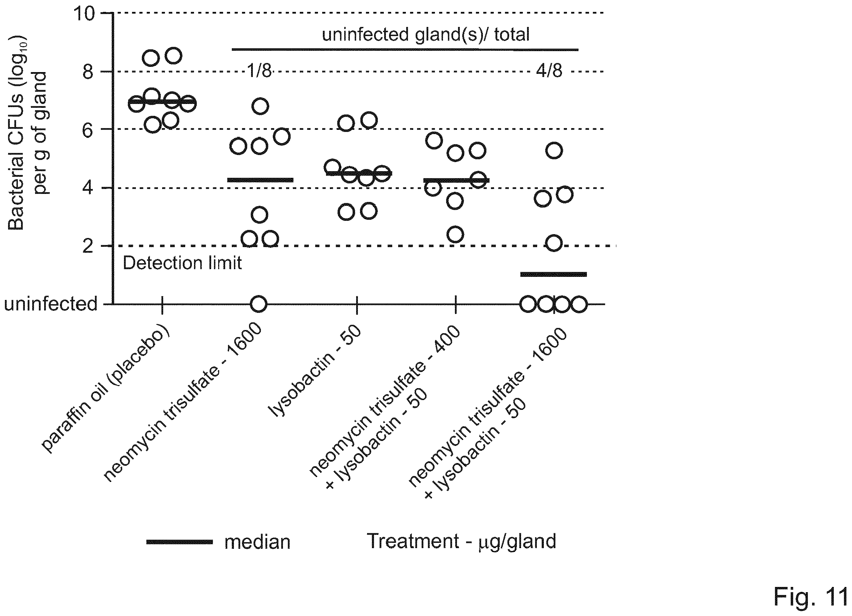

The results are shown in FIG. 11.

Intramammary instillation of 50 µg lysobactin, 1600 µg neomycin trisulfate and the combined 50 µg lysobactin plus 1600 µg neomycin trisulfate reduces the median CFU content by ca. 2.7, 2.5 and 5.9 log₁₀, respectively.

### Example 14: Efficacy of a combination of lysobactin with neomycin trisulfate in an acute mouse mastitis model with S. uberis

Twenty- three lactating CD-1 mice were experimentally infected 10-15 days after birth of the offspring with the mastitis pathogen *Streptococcus uberis* (ATCC 27958) on the left and right fourth mammary gland.

Four hours after infection the animals were treated intrammarily with the test items. The study comprised four treatment groups with four mice each and a control group with seven mice.

The mice of the treatment groups received paraffin formulations of lysobactin at 15 µg/gland or neomycin trisulfate at 1600 µg/gland, or two combinations of both with lysobactin at 15 µg plus neomycin trisulfate at 800 µg or 1600 µg per gland.

Eighteen hours after infection the mice were killed and the mammary glands were removed and bacterial CFU were evaluated microbiologically.

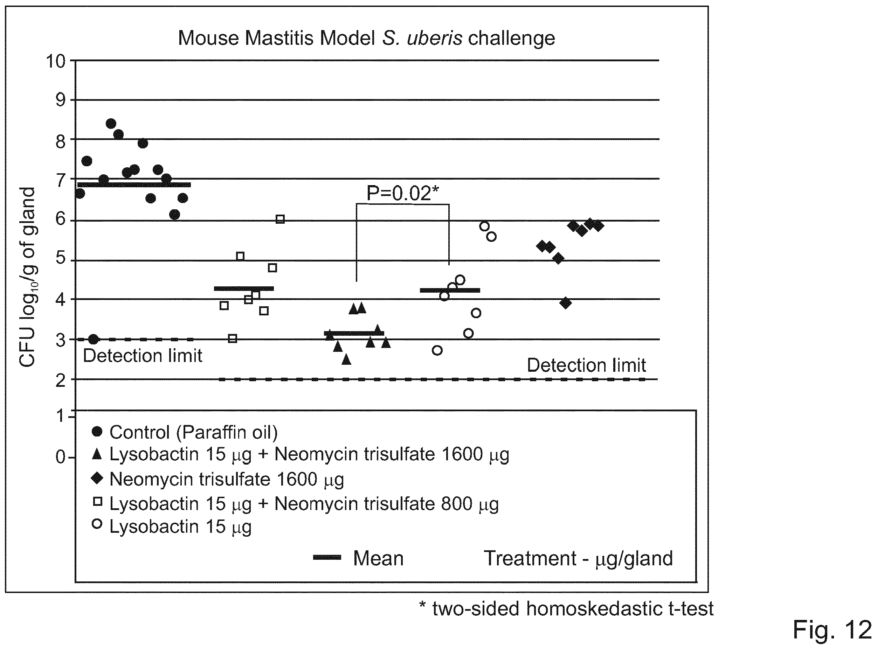

The results are shown in FIG. 12.

The control group receiving paraffin as placebo was in the expected range with CFU of around 7 log₁₀/g of tissue. Intramammary instillation of 15 µg lysobactin, 1600 µg neomycin trisulfate and the combined 15 µg lysobactin plus 1600 µg neomycin trisulfate reduces the mean CFU content by ca. 2.6, 1.5 and 3.8 log₁₀, respectively.

It can be concluded that the lysobactin/neomycin trisulfate combination dosed at 15/1600 µg/gland was superior in its efficacy against *S. uberis* ATCC27958 compared to their single components at the same dosage.

### Example 15: Lysobactin and neomycin trisulfate in vivo cattle data - dairy cow udder infection model with S. aureus

Fifteen healthy lactating dairy cows were experimentally infected with the mastitis pathogen *Staphylococcus aureus* on all four udder quarters. As soon as an udder quarter showed clinical symptoms of mastitis, such as swelling, pain, abnormal milk consistency, it was treated with either lysobactin miglyol based suspension at a dose of 50 mg, or with neomycin trisulfate miglyol based suspension at a dose of 400 mg, or with a combination of both, i.e. 50 mg lysobactin and 400 mg neomycin trisulfate.

Additionally a fourth group of udder quarters received miglyol as negative control. Treatments were randomly assigned to 60 udder quarters in total. Only udder quarters that were bacteriologically positive for the challenge organism immediately prior to treatment (n=49) were eligible for assessment of microbiological and clinical cure. The diseased quarters were treated with these intramammary formulations two times with an interval of 24 hours in between. Udders were clinically examined and milk samples were taken before treatment and several times after that until three weeks after the second administration by study personnel blinded to treatment.

Milk was inspected visually for deviation in its consistency and samples were evaluated for presence of the challenge organism and for somatic cell count to confirm the diagnosis mastitis. Diseased udder quarters were considered microbiologically cured when *Staphylococcus aureus* found in milk samples shortly before treatment could not be isolated from any milk sample taken within the time period between the third and the twenty-first day after the second treatment. Clinical cure was achieved when the local symptoms of mastitis had completely disappeared, and recovery of somatic cell counts (SCC) as parameter of udder inflammation was attained when all counts remained below 200.000 cells per mL of milk in the period mentioned above.

The microbiological cure rate on Day 3 and Day 21 after second treatment was 71% and 14% for lysobactin 50 mg, 100% and 42% for neomycin trisulfate 400 mg, 69% and 54% for the combination of lysobactin 50 mg plus neomycin trisulfate 400 mg, and 10% and 30% for the negative control miglyol, respectively.

The local clinical cure rate on Day 3 and Day 21 after second treatment was 71% and 71% for lysobactin 50 mg, 67% and 100% for neomycin trisulfate 400 mg, 77% and 85% for the combination of lysobactin 50 mg plus neomycin trisulfate 400 mg, and 80% and 70% for the negative control miglyol, respectively.

The SCC recovery rate on Day 3 and Day 21 after second treatment was 35% and 36% for lysobactin 50 mg, 17% and 50% for neomycin trisulfate 400 mg, 46% and 69% for the combination of lysobactin 50 mg plus neomycin trisulfate 400 mg, and 30% and 30% for the negative control miglyol, respectively.

## Claims

1. Combination of lysobactin and at least one aminoglycoside antibiotic for use in a treatment of a non-human mammalian animal against a disease caused by Gram-negative and/or Gram-positive bacteria.

2. Combination for use in a treatment according to claim 1, wherein the mammalian non-human animal is a female animal having an udder.

3. Combination for use in a treatment according to claim 2, wherein the female mammalian non-human animal having an udder is a cow and the disease is bovine mastitis.

4. Combination for use in a treatment according to claim 2 or 3, wherein the combination is administered intramammarily.

5. Combination for use in a treatment according to claim 3 or 4, wherein the bovine mastitis is a clinically manifest bovine mastitis.

6. Combination for use in a treatment according to claim 3 or 4, wherein the bovine mastitis is a subclinical bovine mastitis.

7. Combination for use in a treatment according to one of claims 4 to 6, wherein the lysobactin is provided at a dose of 25 to 1000 mg per udder quarter.

8. Combination for use in a treatment according to one of claims 1 to 7, wherein the at least one aminoglycoside antibiotic is kanamycin or neomycin, preferably the at least one aminoglycoside antibiotic is neomycin.

9. Combination for use in a treatment according to one of claims 3 to 8, wherein the bovine mastitis is caused by *Staphylococcus* bacteria, *Streptococcus* bacteria, *Trueperella* bacteria, *Corynebacterium* bacteria and/or *Escherichia* bacteria.

10. Combination for use in a treatment according to claim 9, wherein the bovine mastitis is caused by *Staphylococcus aureus,* coagulase-negative staphylococci, *Streptococcus uberis, Streptococcus dysgalacticae*, *Streptococcus agalacticae* and/or *E. coli* bacteria.

11. Combination for use in a treatment according to claim 9, wherein the bovine mastitis is caused by *Trueperella pyogenes* and/or *E. coli* bacteria.

12. Combination for use in a treatment according to claim 9, wherein the bovine mastitis is caused by *Corynebacterium bovis* and/or *E. coli* bacteria.

13. Pharmaceutical composition formulated for intramammary administration into bovine mammaries, **characterized in that** the composition comprises lysobactin and at least one aminoglycoside antibiotic, while the at least one aminoglycoside antibiotic is preferably kanamycin or neomycin.

## Patentansprüche

1. Kombination von Lysobactin und mindestens einem Aminoglykosid-Antibiotikum zur Verwendung bei der Behandlung eines nichtmenschlichen Säugetiers gegen eine durch gramnegative und/oder grampositive Bakterien verursachte Krankheit.

2. Kombination zur Verwendung in einer Behandlung nach Anspruch 1, wobei es sich bei dem nichtmenschlichen Säugetier um ein weibliches Tier mit einem Euter handelt.

3. Kombination zur Verwendung in einer Behandlung nach Anspruch 2, wobei es sich bei dem weiblichen nichtmenschlichen Säugetier mit einem Euter um eine Kuh handelt und es sich bei der Krankheit um Rindermastitis handelt.

4. Kombination zur Verwendung in einer Behandlung nach Anspruch 2 oder 3, wobei die Kombination intramammär verabreicht wird.

5. Kombination zur Verwendung in einer Behandlung nach Anspruch 3 oder 4, wobei es sich bei der Rindermastitis um eine klinische manifeste Rindermastitis handelt.

6. Kombination zur Verwendung in einer Behandlung nach Anspruch 3 oder 4, wobei es sich bei der Rindermastitis um eine subklinische Rindermastitis handelt.

7. Kombination zur Verwendung in einer Behandlung nach einem der Ansprüche 4 bis 6, wobei das Lysobactin in einer Dosis von 25 bis 1000 mg pro Euterviertel bereitgestellt wird.

8. Kombination zur Verwendung in einer Behandlung nach einem der Ansprüche 1 bis 7, wobei es sich bei dem mindestens einen Aminoglykosid-Antibiotikum um Kanamycin oder Neomycin handelt, wobei es sich bei dem mindestens einen Aminoglykosid-Antibiotikum vorzugsweise um Neomycin handelt.

9. Kombination zur Verwendung in einer Behandlung nach einem der Ansprüche 3 bis 8, wobei die Rindermastitis durch *Staphylococcus-Bakterien,* Streptococcus-Bakterien, *Trueperella-Bakterien, Corynebacterium*-Bakterien und/oder Escherichia-Bakterien verursacht wird.

10. Kombination zur Verwendung in einer Behandlung nach Anspruch 9, wobei die Rindermastitis durch *Staphylococcus aureus,* koagulasenegative Staphylokokken, *Streptococcus uberis, Streptococcus dysgalacticae*, *Streptococcus agalacticae* und/oder E. coli-Bakterien verursacht wird.

11. Kombination zur Verwendung in einer Behandlung nach Anspruch 9, wobei die Rindermastitis durch *Trueperella pyogenes* und/oder *E. coli*-Bakterien verursacht wird.

12. Kombination zur Verwendung in einer Behandlung nach Anspruch 9, wobei die Rindermastitis durch *Corynebacterium bovis* und/oder *E. coli*-Bakterien verursacht wird.

13. Pharmazeutische Zusammensetzung, formuliert für die intramammäre Verabreichung in Rindereuter, **dadurch gekennzeichnet, dass** die Zusammensetzung Lysobactin und mindestens ein Aminoglykosid-Antibiotikum umfasst, wobei es sich bei dem mindestens einen Aminoglykosid-Antibiotikum vorzugsweise um Kanamycin oder Neomycin handelt.

## Revendications

1. Combinaison de lysobactine et d'au moins un antibiotique aminoglycoside pour une utilisation dans un traitement d'un animal mammifère non humain contre une maladie causée par des bactéries Gram négatif et/ou Gram positif.

2. Combinaison pour une utilisation dans un traitement selon la revendication 1, où l'animal mammifère non humain est un animal femelle ayant une mamelle.

3. Combinaison pour une utilisation dans un traitement selon la revendication 2, où l'animal mammifère non humain femelle ayant une mamelle est une vache et la maladie est la mammite bovine.

4. Combinaison pour une utilisation dans un traitement selon la revendication 2 ou 3, où la combinaison est administrée par voie intramammaire.

5. Combinaison pour une utilisation dans un traitement selon la revendication 3 ou 4, où la mammite bovine est une mammite bovine cliniquement manifeste.

6. Combinaison pour une utilisation dans un traitement selon la revendication 3 ou 4, où la mammite bovine est une mammite bovine subclinique.

7. Combinaison pour une utilisation dans un traitement selon l'une des revendications 4 à 6, où la lysobactine est fournie à une dose de 25 à 1000 mg par quartier de pis.

8. Combinaison pour une utilisation dans un traitement selon l'une des revendications 1 à 7, dans laquelle l'au moins un antibiotique aminoglycoside est la kanamycine ou la néomycine, de préférence l'au moins un antibiotique aminoglycoside est la néomycine.

9. Combinaison pour une utilisation dans un traitement selon l'une des revendications 3 à 8, où la mammite bovine est causée par des bactéries *Staphylococcus*, des bactéries *Streptococcus,* des bactéries *Trueperella*, des bactéries *Corynebacterium* et/ou des bactéries *Escherichia.*

10. Combinaison pour une utilisation dans un traitement selon la revendication 9, où la mammite bovine est causée par des bactéries *Staphylococcus aureus,* staphylocoques à coagulase négative, *Streptococcus uberis, Streptococcus dysgalacticae*, *Streptococcus agalacticae* et/ou *E. coli.*

11. Combinaison pour une utilisation dans un traitement selon la revendication 9, où la mammite bovine est causée par des bactéries *Trueperella pyogenes* et/ou *E. coli.*

12. Combinaison pour une utilisation dans un traitement selon la revendication 9, où la mammite bovine est causée par des bactéries *Corynebacterium bovis* et/ou *E. coli.*

13. Composition pharmaceutique formulée pour une administration intramammaire dans des mamelles bovines, **caractérisée en ce que** la composition comprend de la lysobactine et au moins un antibiotique aminoglycoside, tandis que l'au moins un antibiotique aminoglycoside est de préférence la kanamycine ou la néomycine.
